# EUROPEAN PATENT APPLICATION

(11) **EP 4 691 374 A1**
(43) Date of publication of application: **11.02.2026**
(21) Application number: 24779345.8
(22) Date of filing: 11.03.2024
(51) Int. Cl.: A61B 8/14

(54) **ULTRASONIC DIAGNOSTIC DEVICE AND METHOD FOR CONTROLLING ULTRASONIC DIAGNOSTIC DEVICE**

(30) Priority: 29.03.2023 JP 2023053096
(71) Applicant: FUJIFILM Corporation, Tokyo 106-8620 (JP)
(72) Inventor: KATO, Noriji, Tokyo 106-8620 (JP)
(74) Representative: Klunker IP Patentanwälte PartG mbB
(86) International application number: PCT/JP2024/009297
(87) International publication number: WO 2024/203214

(57) **Abstract**

An ultrasound diagnostic apparatus includes: an image acquisition unit (36) that performs a scan using an ultrasound probe (1) to acquire ultrasound images of a breast; a mammary gland region extraction unit (25) that extracts a mammary gland region from the ultrasound images; a thickness calculation unit (26) that calculates a thickness of the mammary gland region in a depth direction; a lesion detection unit (27) that detects a suspected lesion region for the ultrasound images; a frame selection unit (29) that selects, as an evaluation target frame group, at least frames which exclude a frame in which the suspected lesion region is detected, and in which the thickness of the mammary gland region is equal to or greater than a thickness threshold value, among a plurality of frames; and an evaluation unit (32) that performs a glandular tissue component evaluation on the ultrasound image of the evaluation target frame group.

## Description

### BACKGROUND OF THE INVENTION

### 1. Field of the Invention

The present invention relates to an ultrasound diagnostic apparatus used for an examination of a breast of a subject and a method of controlling the ultrasound diagnostic apparatus.

### 2. Description of the Related Art

In related art, in the medical field, an ultrasound diagnostic apparatus using ultrasound images is put into practical use. In general, the ultrasound diagnostic apparatus comprises an ultrasound probe provided with a transducer array and an apparatus body connected to the ultrasound probe, in which an ultrasound beam is transmitted from the ultrasound probe toward a subject, an ultrasound echo from the subject is received by the ultrasound probe, and a reception signal is electrically processed to generate the ultrasound image.

A composition of a fat tissue and a mammary gland tissue in a breast varies depending on a person, but an anatomical structure of the breast is common, and a primary lactiferous duct branches into extralobular ducts, which in turn connect to numerous lobules, in the mammary gland tissue. Stroma is present around the lobules, and mammary gland tissue is composed of the lobules together with the stroma.

It is known that two types of stroma exist around the lobules, that is, perilobular stroma and edematous stroma. The perilobular stroma exists along a structure from the lobule to the mammary duct, and includes many collagen fibers. Meanwhile, the edematous stroma fills the spaces between the perilobular stroma, is rich in extracellular matrix, with a mixture of collagen fibers and fat, and contains fewer collagen fibers as compared to the perilobular stroma.

In recent years, the concept of individualized risk management for patients has become widespread, but it is known that a ratio of the mammary gland region within the breast, especially a high-density mammary gland, is a risk factor for cancer. The ratio of the mammary gland region in the breast can be measured by using a mammography apparatus.

In Su Hyun Lee et al. "Glandular Tissue Component and Breast Cancer Risk in Mammographically Dense Breasts at Screening Breast US", Radiology, Volume 301, October 1, 2021, it is reported that a cancer is likely to occur in a case in which a ratio of a glandular tissue component (GTC) region including mammary ducts, lobules, and perilobular stroma in the mammary gland region is high even though the mammary gland region is almost the same. That is, in addition to the ratio of the mammary gland region in the breast, a ratio of the GTC region in the mammary gland region may be a risk factor. This means a higher risk in a patient with less advanced atrophy of the lobule.

However, in the mammography apparatus, the perilobular stroma and the edematous stroma cannot be distinguished from each other, and the entire mammary gland tissue is observed as whitish, and as a result, the ratio of the GTC region in the mammary gland region cannot be measured.

### SUMMARY OF THE INVENTION

In general, in a case in which the breast of the subject is examined using the ultrasound diagnostic apparatus in a so-called health checkup or the like, there is often work of scanning the entire breast using the ultrasound probe in order to search for a suspected lesion region which is a region suspected to be a lesion. As disclosed in Su Hyun Lee et al. "Glandular Tissue Component and Breast Cancer Risk in Mammographically Dense Breasts at Screening Breast US", Radiology, Volume 301, October 1, 2021, it is necessary to perform work of searching for a cross section in which the mammary gland region is appropriately depicted in addition to work of searching for a cross section in which the suspected lesion region is appropriately depicted in a case in which the GTC region is evaluated, and thus it is difficult for the user such as a doctor to easily perform consideration of the risk of breast cancer based on the GTC region, which may be a heavy burden.

The present invention has been made in order to solve such a problem in the related art, and an object of the present invention is to provide an ultrasound diagnostic apparatus that enables a user to easily consider a risk of breast cancer based on a GTC region.

It is possible to achieve the above-described object with the following configurations.
[1] An ultrasound diagnostic apparatus comprising: an ultrasound probe; an image acquisition unit that performs a scan using the ultrasound probe to continuously acquire ultrasound images of a plurality of frames in which a breast of a subject is imaged; a mammary gland region extraction unit that extracts a mammary gland region from each of the ultrasound images of the plurality of frames and that calculates an area of the mammary gland region; a thickness calculation unit that calculates a thickness of the mammary gland region in a depth direction, which is extracted by the mammary gland region extraction unit, for each of the ultrasound images of the plurality of frames; a lesion detection unit that detects a suspected lesion region for each of the ultrasound images of the plurality of frames; a frame selection unit that selects, as an evaluation target frame group, at least frames which exclude a frame in which the suspected lesion region is detected by the lesion detection unit, and in which the thickness of the mammary gland region in the depth direction, which is calculated by the thickness calculation unit, is equal to or greater than a predetermined thickness threshold value, among the plurality of frames; and an evaluation unit that performs a glandular tissue component evaluation on the ultrasound image of each frame of the evaluation target frame group.
[2] The ultrasound diagnostic apparatus according to [1], further comprising: a shadow detection unit that detects a shadow for each of the ultrasound images of the plurality of frames, in which the frame selection unit selects, as the evaluation target frame group, frames which exclude the frame in which the suspected lesion region is detected by the lesion detection unit and a frame in which the shadow is detected by the shadow detection unit, and in which the thickness of the mammary gland region in the depth direction, which is calculated by the thickness calculation unit, is equal to or greater than the predetermined thickness threshold value.
[3] The ultrasound diagnostic apparatus according to [1], further comprising: an evaluation result memory that stores an evaluation result obtained by the evaluation unit; a monitor; and a display control unit that displays the ultrasound image and the evaluation result on the monitor.
[4] The ultrasound diagnostic apparatus according to [3], further comprising: a low-brightness region extraction unit that extracts a low-brightness region having brightness equal to or less than a predetermined brightness threshold value from the mammary gland region extracted by the mammary gland region extraction unit for the ultrasound image of the evaluation target frame group; and a glandular tissue component ratio calculation unit that calculates a glandular tissue component ratio based on the area of the mammary gland region extracted by the mammary gland region extraction unit for the ultrasound image of the evaluation target frame group and an area of the low-brightness region extracted by the low-brightness region extraction unit, in which the evaluation unit performs the glandular tissue component evaluation based on the glandular tissue component ratio calculated by the glandular tissue component ratio calculation unit.
[5] The ultrasound diagnostic apparatus according to [4], further comprising: a frame group readjustment unit that, in a case in which a difference between the glandular tissue component ratio in one frame in the evaluation target frame group and the glandular tissue component ratio in a frame immediately preceding the one frame is equal to or less than a predetermined ratio threshold value, readjusts the evaluation target frame group such that the one frame is excluded, in which the evaluation unit stores the glandular tissue component ratio in the evaluation target frame group readjusted by the frame group readjustment unit in the evaluation result memory as a result of the glandular tissue component evaluation, and displays the glandular tissue component ratio on the monitor.
[6] The ultrasound diagnostic apparatus according to [4], further comprising: a ratio memory that stores the glandular tissue component ratio calculated by the glandular tissue component ratio calculation unit; a histogram creation unit that creates a histogram of the glandular tissue component ratio in a plurality of frames in the evaluation target frame group stored in the ratio memory; and a frame group readjustment unit that readjusts the evaluation target frame group such that a frame corresponding to the glandular tissue component ratio that appears with a higher frequency than a predetermined distribution in the histogram is excluded, in which the evaluation unit stores the glandular tissue component ratio in the evaluation target frame group readjusted by the frame group readjustment unit in the evaluation result memory as a result of the glandular tissue component evaluation, and displays the glandular tissue component ratio on the monitor.
[7] The ultrasound diagnostic apparatus according to any one of [1] to [6], in which the frame selection unit excludes the frames at regular intervals in time series from the plurality of frames, and selects the evaluation target frame group from remaining frame groups.
[8] A method of controlling an ultrasound diagnostic apparatus, the method comprising: performing a scan using an ultrasound probe to continuously acquire ultrasound images of a plurality of frames in which a breast of a subject is imaged; extracting a mammary gland region from each of the ultrasound images of the plurality of frames; calculating a thickness of the extracted mammary gland region in a depth direction for each of the ultrasound images of the plurality of frames; detecting a suspected lesion region for each of the ultrasound images of the plurality of frames; selecting, as an evaluation target frame group, at least frames which exclude a frame in which the suspected lesion region is detected, and in which the calculated thickness of the mammary gland region in the depth direction is equal to or greater than a predetermined thickness threshold value, among the plurality of frames; and performing a glandular tissue component evaluation on the ultrasound image of each frame of the evaluation target frame group.

According to the aspects of the present invention, the ultrasound diagnostic apparatus comprises the ultrasound probe, the image acquisition unit that performs the scan using the ultrasound probe to continuously acquire the ultrasound images of the plurality of frames in which the breast of the subject is imaged, the mammary gland region extraction unit that extracts the mammary gland region from each of the ultrasound images of the plurality of frames and that calculates the area of the mammary gland region, the thickness calculation unit that calculates the thickness of the mammary gland region in the depth direction, which is extracted by the mammary gland region extraction unit, for each of the ultrasound images of the plurality of frames, the lesion detection unit that detects the suspected lesion region for each of the ultrasound images of the plurality of frames, the frame selection unit that selects, as the evaluation target frame group, at least frames which exclude the frame in which the suspected lesion region is detected by the lesion detection unit, and in which the thickness of the mammary gland region in the depth direction, which is calculated by the thickness calculation unit, is equal to or greater than the predetermined thickness threshold value, among the plurality of frames, and the evaluation unit that performs the glandular tissue component evaluation on the ultrasound image of each frame of the evaluation target frame group, so that the evaluation can be easily performed to allow the user to easily consider the risk of breast cancer based on the glandular tissue component (GTC) region.

### BRIEF DESCRIPTION OF THE DRAWINGS

FIG. 1 is a block diagram showing a configuration of an ultrasound diagnostic apparatus according to Embodiment 1 of the present invention.
FIG. 2 is a block diagram showing an internal configuration of a transmission and reception circuit according to Embodiment 1.
FIG. 3 is a block diagram showing an internal configuration of an image generation unit according to Embodiment 1.
FIG. 4 is a diagram showing an ultrasound image obtained by imaging a mammary gland region of a subject.
FIG. 5 is a diagram showing an ultrasound image including a mammary gland region in which a GTC region is imaged.
FIG. 6 is a flowchart showing an operation in Embodiment 1.
FIG. 7 is a block diagram showing a configuration of an ultrasound diagnostic apparatus according to Embodiment 2 of the present invention.
FIG. 8 is a block diagram showing a configuration of an ultrasound diagnostic apparatus according to Embodiment 3 of the present invention.
FIG. 9 is a block diagram showing a configuration of an ultrasound diagnostic apparatus according to Embodiment 4 of the present invention.
FIG. 10 is a block diagram showing a configuration of an ultrasound diagnostic apparatus according to Embodiment 5 of the present invention.
FIG. 11 is a diagram showing an example of a histogram of GTC ratios in a plurality of frames in an evaluation target frame group.

### DESCRIPTION OF THE PREFERRED EMBODIMENTS

Hereinafter, embodiments of the invention will be described with reference to the accompanying drawings.

The following configuration requirements are described based on a representative embodiment of the present invention, but the present invention is not limited to such an embodiment.

In addition, the present specification, a numerical range represented by "to" means a range including numerical values described before and after "to", both ends inclusive, as a lower limit value and an upper limit value.

In the present specification, "same" and "identical" include an error range that is generally allowed in the technical field.

### [Embodiment 1]

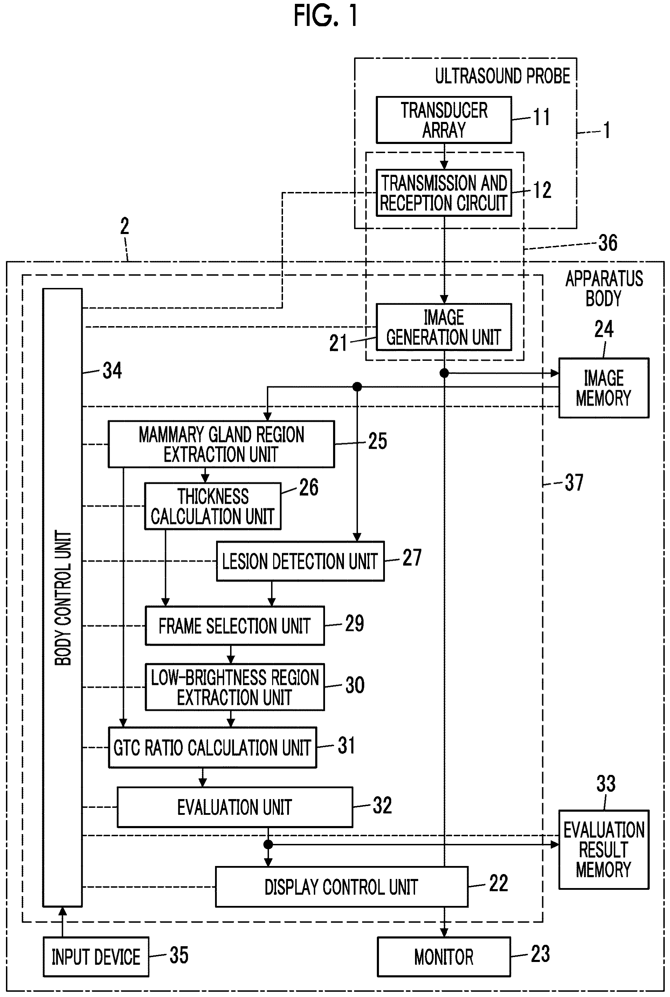

FIG. 1 shows a configuration of an ultrasound diagnostic apparatus according to the embodiment of the present invention. The ultrasound diagnostic apparatus comprises an ultrasound probe 1 and an apparatus body 2. The ultrasound probe 1 and the apparatus body 2 are wired-connected to each other through a cable (not shown).

The ultrasound probe 1 includes a transducer array 11 and a transmission and reception circuit 12 connected to the transducer array 11.

The apparatus body 2 includes an image generation unit 21 connected to the transmission and reception circuit 12 of the ultrasound probe 1, a display control unit 22 and a monitor 23 are connected sequentially to the image generation unit 21, and an image memory 24 is connected to the image generation unit 21. A mammary gland region extraction unit 25 is connected to the image memory 24. A thickness calculation unit 26 is connected to the mammary gland region extraction unit 25. Further, a lesion detection unit 27 is connected to the image memory 24. A frame selection unit 29 is connected to the thickness calculation unit 26 and the lesion detection unit 27. A low-brightness region extraction unit 30 is connected to the frame selection unit 29. Further, a glandular tissue component (GTC) ratio calculation unit 31 is connected to the mammary gland region extraction unit 25 and the low-brightness region extraction unit 30. An evaluation unit 32 is connected to the GTC ratio calculation unit 31. The display control unit 22 and an evaluation result memory 33 are connected to the evaluation unit 32.

In addition, a body control unit 34 is connected to the transmission and reception circuit 12, the image generation unit 21, the display control unit 22, the image memory 24, the mammary gland region extraction unit 25, the thickness calculation unit 26, the lesion detection unit 27, the frame selection unit 29, the low-brightness region extraction unit 30, the GTC ratio calculation unit 31, the evaluation unit 32, and the evaluation result memory 33. An input device 35 is connected to the body control unit 34. The transmission and reception circuit 12 and the image generation unit 21 constitute an image acquisition unit 36. The image generation unit 21, the display control unit 22, the mammary gland region extraction unit 25, the thickness calculation unit 26, the lesion detection unit 27, the frame selection unit 29, the low-brightness region extraction unit 30, the GTC ratio calculation unit 31, the evaluation unit 32, and the body control unit 34 constitute a processor 37 for the apparatus body 2.

The transducer array 11 of the ultrasound probe 1 includes a plurality of ultrasound transducers arranged in a one-dimensional or two-dimensional manner. Each of these transducers transmits an ultrasound wave in response to a drive signal supplied from the transmission and reception circuit 12, receives a reflected wave from a subject, and outputs an analog reception signal. Each transducer is formed by, for example, forming electrodes on both ends of a piezoelectric body consisting of a piezoelectric single crystal represented by lead zirconate titanate (PZT), a polymeric piezoelectric element represented by poly vinylidene di fluoride (PVDF), or a piezoelectric single crystal represented by a lead magnesium niobate-lead titanate (PMN-PT) solid solution.

The image acquisition unit 36 composed of the transmission and reception circuit 12 and the image generation unit 21 continuously performs a scan using the ultrasound probe 1 to acquire ultrasound images of a plurality of frames in which the breast of the subject is imaged.

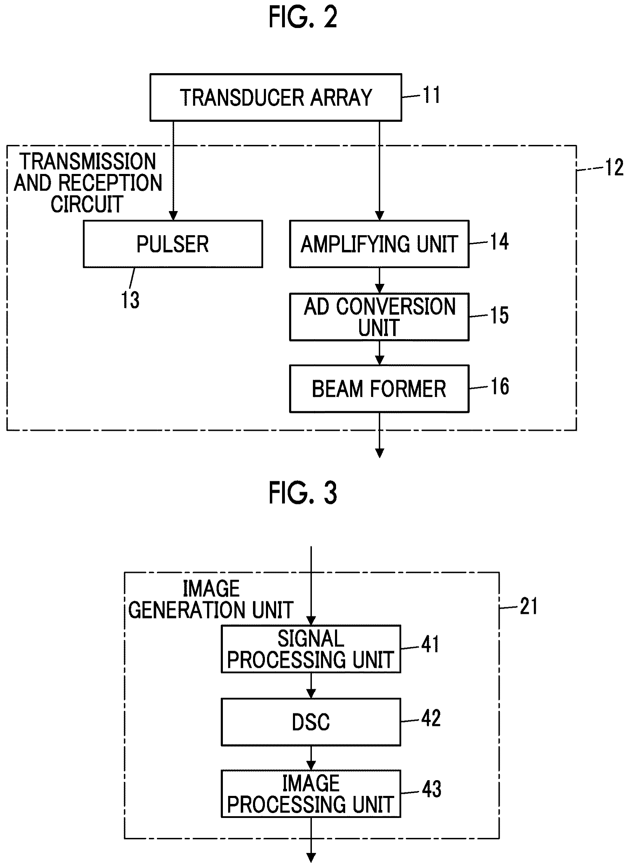

The transmission and reception circuit 12 transmits the ultrasound wave from the transducer array 11 and generates a sound ray signal based on the reception signal acquired by the transducer array 11, under the control of the body control unit 34. The transmission and reception circuit 12 includes, as shown in FIG. 2, a pulser 13 connected to the transducer array 11, and an amplifying unit 14, an analog-to-digital (AD) conversion unit 15, and a beam former 16 which are sequentially connected in series to the transducer array 11.

The pulser 13 includes, for example, a plurality of pulse generators, adjusts a delay amount of each drive signal based on a transmission delay pattern selected in accordance with a control signal from the body control unit 34 such that ultrasound waves to be transmitted from the plurality of transducers of the transducer array 11 form a ultrasound beam, and supplies the drive signal of which the delay amount has been adjusted, to the plurality of transducers. As described above, in a case in which a pulsed or continuous wave voltage is applied to the electrodes of the transducers of the transducer array 11, the piezoelectric body expands and contracts to generate a pulsed or continuous wave ultrasound wave from each transducer, and the ultrasound beam is formed from the combined wave of these ultrasound waves.

The transmitted ultrasound beam is, for example, reflected by a target such as a part of the subject, and an ultrasound echo propagates toward the transducer array 11 of the ultrasound probe 1. The ultrasound echo propagating toward the transducer array 11 in this manner is received by each of the transducers constituting the transducer array 11. In such a case, each transducer constituting the transducer array 11 expands and contracts by receiving the propagating ultrasound echo to generate the reception signal that is an electric signal, and outputs the reception signal to the amplifying unit 14.

The amplifying unit 14 amplifies the signal input from each of the transducers constituting the transducer array 11 and transmits the amplified signal to the AD conversion unit 15. The AD conversion unit 15 converts the signal transmitted from the amplifying unit 14 into digital reception data, and transmits the reception data to the beam former 16. The beam former 16 performs so-called reception focus processing by giving and adding delay with respect to each reception data converted by the AD conversion unit 15, in accordance with a sound velocity or a sound velocity distribution set based on a reception delay pattern selected according to a control signal from the body control unit 34. By the reception focus processing, a sound ray signal is acquired in which each piece of the reception data converted by the AD conversion unit 15 is phased and added and the focus of the ultrasound echo is narrowed.

The image generation unit 21 of the apparatus body 2 has, as shown in FIG. 3, a configuration in which a signal processing unit 41, a digital scan converter (DSC) 42, and an image processing unit 43 are sequentially connected in series.

The signal processing unit 41 performs, on the sound ray signal transmitted from the transmission and reception circuit 12 of the ultrasound probe 1, correction of attenuation caused by a distance in accordance with a depth of a reflection position of the ultrasound wave and then performs envelope detection processing, and thereby generates an ultrasound image signal (B-mode image signal), which is tomographic image information related to tissues in the subject.

The DSC 42 converts (raster-converts) the ultrasound image signal generated by the signal processing unit 41 into an image signal in accordance with a normal television signal scanning method.

The image processing unit 43 performs various types of necessary image processing, such as gradation processing, on the ultrasound image signal input from the DSC 42, and then outputs the signal representing the ultrasound image to the display control unit 22 and the image memory 24. The signal representing the ultrasound image generated by the image generation unit 21 in this way will be simply referred to as the ultrasound image. The image generation unit 21 can also output the ultrasound image signal before being processed by the DSC 42 or the ultrasound image signal immediately after being processed by the DSC 42 to the image memory 24. In such a case, the image generation unit 21 can generate the ultrasound image by reading out these signals from the image memory 24 and performing processing using the DSC 42 or the image processing unit 43.

The image acquisition unit 36, which is configured by the transmission and reception circuit 12 and the image generation unit 21, acquires the ultrasound images of the plurality of frames as described above in a state in which the ultrasound probe 1 is moved on the breast of the subject by a user such as a doctor.

The image memory 24 is a memory that stores the ultrasound image generated by the image generation unit 21 under the control of the body control unit 34. For example, the image memory 24 can store a plurality of frames of ultrasound images generated by the image generation unit 21 in correspondence with diagnosis on a mammary gland region of a breast of the subject.

As the image memory 24, for example, a recording medium such as a flash memory, a hard disc drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), or a universal serial bus memory (USB memory), can be used.

The mammary gland region extraction unit 25 detects a breast region of the subject from the ultrasound images of the plurality of frames read out from the image memory 24, and extracts the mammary gland region from the detected breast region.

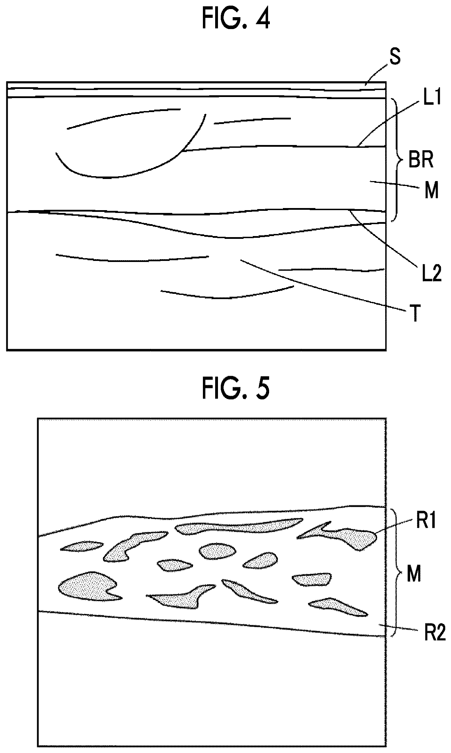

FIG. 4 shows an example of an ultrasound image U in which the breast of the subject is imaged. The ultrasound image U is a tomographic image captured by bringing a distal end of the ultrasound probe 1 into contact with the breast of the subject, in which a skin S of the subject is shown in an upper end of the ultrasound image U representing a shallowest portion, and a pectoralis major T is shown in a lower portion of the ultrasound image U representing a deeper portion. The mammary gland region extraction unit 25 can recognize a skin S and a pectoralis major T from the ultrasound image U and detect a deep region between the skin S and the pectoralis major T as a breast region BR.

As shown in FIG. 4, the mammary gland region extraction unit 25 can recognize a front boundary line L1 located on a shallower side and a rear boundary line L2 located on a deeper side in the detected breast region BR, and can extract a deep region between the front boundary line L1 and the rear boundary line L2 as a mammary gland region M.

In order to detect the breast region BR and to extract the mammary gland region M described above, the mammary gland region extraction unit 25 can perform image recognition using at least one of template matching, an image analysis technique using a feature value, such as adaptive boosting (AdaBoost), support vector machine (SVM), or scale-invariant feature transform (SIFT), or a determination model that has been trained by using a machine learning technique such as deep learning.

The determination model is a trained model that has learned the breast region BR and the mammary gland region M (segmentation) of the breast region BR in a training ultrasound image obtained by imaging the breast.

The thickness calculation unit 26 calculates a thickness of a mammary gland region M in a depth direction extracted by the mammary gland region extraction unit 25 for each of the ultrasound images U of the plurality of frames. For example, the thickness calculation unit 26 can calculate a maximum thickness of the mammary gland region M in the depth direction as the thickness of the mammary gland region M in the depth direction.

The lesion detection unit 27 detects a suspected lesion region for each of the ultrasound images U of the plurality of frames. The suspected lesion region refers to a region in which a lesion including a so-called tumor is suspected, in the mammary gland region M. The lesion detection unit 27 can detect the suspected lesion region by using, for example, at least one of template matching, an image analysis technique using a feature value, such as AdaBoost, SVM, or SIFT, or a determination model that has been trained by using a machine learning technique such as deep learning. The determination model used here is a trained model that has learned a plurality of lesion parts in the ultrasound image U in which the breast region BR is imaged.

The frame selection unit 29 has a predetermined thickness threshold value for the thickness of the mammary gland region M in the depth direction, which is calculated by the thickness calculation unit 26, and selects, as an evaluation target frame group, at least frames which exclude a frame in which the suspected lesion region is detected by the lesion detection unit 27, and in which the thickness of the mammary gland region M in the depth direction, which is calculated by the thickness calculation unit 26, is equal to or greater than the thickness threshold value, among the ultrasound images U of the plurality of frames generated by the image generation unit 21.

The low-brightness region extraction unit 30 has a predetermined brightness threshold value related to the ultrasound image U, and extracts, as shown in FIG. 5, a low-brightness region R1 having brightness equal to or less than the brightness threshold value from the mammary gland region M extracted by the mammary gland region extraction unit 25 for the ultrasound image U of the evaluation target frame group selected by the frame selection unit 29.

Here, the low-brightness region R1 is a region mainly composed of the GTC regions. The GTC region consists of mammary ducts, lobules, and perilobular stroma in the mammary gland region M, and edematous stroma R2 fills a space between the perilobular stroma. Since the edematous stroma R2 is rich in extracellular matrix and contains coexisting fat, in a case in which the mammary gland region M is observed using the ultrasound image U, the edematous stroma R2 has a high echo level (high-echo) and appears bright. On the other hand, the mammary ducts, the lobules, and the perilobular stroma constituting the GTC region have relatively low echo levels (low-echo), and have lower brightness than the edematous stroma R2.

In general, since the suspected lesion region has a relatively low-echo level like the GTC region, the brightness of the suspected lesion region is lower than that of the edematous stroma R2. In this way, normally, both the GTC region and the suspected lesion region are depicted as the low-brightness region R1 in the ultrasound image U, but since the evaluation target frame group excludes the frame in which the suspected lesion region is detected by the lesion detection unit 27, the suspected lesion region is effectively excluded in the low-brightness region R1 in the ultrasound image U of the evaluation target frame group.

In addition, in a case in which the mammary gland region M in the ultrasound image U does not have a thickness equal to or greater than a certain thickness in the depth direction, the low-brightness region R1 in the mammary gland region M is depicted to be very thin, and thus the low-brightness region R1 may not be normally detected. The mammary gland region M in the ultrasound image U of the evaluation target frame group has a thickness equal to or greater than the thickness threshold value in the depth direction, and thus the low-brightness region extraction unit 30 can normally detect the low-brightness region R1.

The low-brightness region extraction unit 30 can extract, for example, a pixel at which brightness is equal to or less than a predetermined brightness threshold value from the mammary gland region M extracted by the mammary gland region extraction unit 25, and calculate an area occupied by the extracted pixel as the area of the low-brightness region R1 for each frame.

In addition, the low-brightness region extraction unit 30 can also binarize the mammary gland region M extracted by the mammary gland region extraction unit 25 based on the predetermined brightness threshold value, and then use an algorithm such as a so-called watershed method, to extract the low-brightness region R1.

In addition, the low-brightness region extraction unit 30 can also extract the low-brightness region R1 by using a determination model that has been trained by using a machine learning technique such as deep learning. As the determination model, for example, a trained model, which has learned the low-brightness region R1 in the mammary gland region M in the training ultrasound image in which the breast is imaged, is used.

A predetermined constant value can be used as the brightness threshold value.

In addition, the low-brightness region extraction unit 30 may perform edge detection on the low-brightness region R1 in the ultrasound image U by image analysis, and automatically calculate the brightness threshold value based on a change in the brightness value in the detected edge portion, that is, a change in the brightness value of a plurality of pixels from the inside to the outside of the low-brightness region R1. In this way, the brightness threshold value suitable for the ultrasound image U to be subjected to the image analysis can be automatically set.

Further, the low-brightness region extraction unit 30 can also set a value input by the user via the input device 35 as the brightness threshold value used for the binarization of the mammary gland region M. In such a case, the low-brightness region extraction unit 30 can generate a binarized image of the mammary gland region M using an initial value of the brightness threshold value, and create a histogram of the brightness of the mammary gland region M in the ultrasound image U. Further, the user can input an updated value of the brightness threshold value while referring to, for example, the binarized image generated using the initial value, the histogram of the brightness, and the ultrasound image U. In a case in which the brightness threshold value is input by the user in this way, the low-brightness region extraction unit 30 can update the binarized image using the brightness threshold value input by the user.

The GTC ratio calculation unit 31 calculates the GTC ratio by a ratio of the area of the low-brightness region R1 extracted by the low-brightness region extraction unit 30 to the area of the mammary gland region M extracted by the mammary gland region extraction unit 25 for each ultrasound image U of the evaluation target frame group selected by the frame selection unit 29. Since the GTC ratio calculation unit 31 calculates the GTC ratio based on the ultrasound image U of the evaluation target frame group, the GTC ratio calculation unit 31 can accurately calculate the GTC ratio.

The evaluation unit 32 performs the GTC evaluation on the ultrasound image U of each frame of the evaluation target frame group, based on the GTC ratio calculated by the GTC ratio calculation unit 31.

It is generally known that the lobule atrophies with age, but there are research results that a risk of breast cancer is high in patients in whom the lobule does not atrophy, as disclosed in, for example, "Su Hyun Lee et al. "Glandular Tissue Component and Breast Cancer Risk in Mammographically Dense Breasts at Screening Breast US", Radiology, Volume 301, October 1, 2021".

The GTC ratio indicates the degree of progression of the atrophy of the lobule and can be used as a material for determining the risk of breast cancer. Therefore, the evaluation unit 32 can use, for example, an average value, a median value, or a sum value of the GTC ratio calculated in each of the evaluation target frame groups as the evaluation result of the GTC evaluation. The user, such as the doctor, can determine that the lower the value of the GTC ratio, the higher the risk of breast cancer.

Further, for example, the evaluation unit 32 stores a predetermined breast cancer risk function for calculating a higher breast cancer risk value as the GTC ratio is lower, and can use the GTC ratio and the breast cancer risk value calculated based on the breast cancer risk function as the evaluation result of the GTC evaluation. The user can determine that the higher the breast cancer risk value, the higher the risk of breast cancer.

In addition, the evaluation unit 32 can determine the category of the GTC region based on the GTC ratio, and use the category as the evaluation result of the GTC evaluation.

The evaluation unit 32 can output, as the evaluation result, any one of a plurality of predetermined categories, for example, any one of two categories of Low and High as the category of the GTC region. Low indicates that the lobule atrophy has not progressed as much as in High. Further, the evaluation unit 32 can also output, for example, any one of four categories of Minimal, Mild, Moderate, and Marked as the category of the GTC region. Mild indicates that the atrophy of the lobule is not more advanced than that in Minimal, Moderate indicates that the atrophy of the lobule is not more advanced than that in Mild, and Marked indicates that the atrophy of the lobule is not more advanced than that in Moderate.

In general, in a case in which the breast of the subject is examined using the ultrasound diagnostic apparatus in a so-called health checkup or the like, the user such as the doctor often scans the entire breast using the ultrasound probe 1 in order to search for the suspected lesion region. In a case in which the evaluation of the GTC region is to be performed, it is necessary to perform work of searching for the cross section in which the mammary gland region M including the GTC region is appropriately depicted in addition to work of searching for the cross section in which the suspected lesion region is appropriately depicted, so that there is a case in which the burden on the user such as the doctor is large, and it is difficult to easily perform the evaluation of the GTC region.

In the ultrasound diagnostic apparatus according to Embodiment 1 of the present invention, the user does not have to perform the work of evaluating the GTC region, for example, only by scanning the breast of the subject in order to search for the suspected lesion region, the frame selection unit 29 automatically selects the evaluation target frame group suitable for the evaluation of the GTC region, and the evaluation unit 32 automatically performs the evaluation of the GTC region based on the ultrasound image U of the evaluation target frame group, so that it is possible to easily perform the consideration of the risk of breast cancer based on the GTC region.

The evaluation result memory 33 stores the evaluation result of the risk of breast cancer by the evaluation unit 32. The user can, for example, read out the evaluation result via the input device 35 after the examination of the subject, and consider the risk of breast cancer in the breast of the subject based on the evaluation result. As the evaluation result memory 33, for example, recording media such as a flash memory, an HDD, an SSD, an FD, an MO disc, an MT, an RAM, a CD, a DVD, an SD card, and an USB memory can be used.

The display control unit 22 performs predetermined processing on the ultrasound image U sent from the image generation unit 21, the evaluation result of the GTC region by the evaluation unit 32, and the like under the control of the body control unit 34, and displays the ultrasound image U, the evaluation result of the GTC region, and the like on the monitor 23.

The monitor 23 displays the ultrasound image U and the like under the control of the display control unit 22, and includes, for example, a display device such as a liquid crystal display (LCD) or an organic electroluminescence display (organic EL display).

The body control unit 34 controls each unit of the apparatus body 2 and the transmission and reception circuit 12 of the ultrasound probe 1 based on a control program or the like, which is stored in advance.

The input device 35 is an input device used by the user to perform an input operation, and is configured by, for example, a device such as a keyboard, a mouse, a trackball, a touchpad, and a touch sensor disposed in a state of being superimposed on the monitor 23.

The processor 37 including the image generation unit 21, the display control unit 22, the mammary gland region extraction unit 25, the thickness calculation unit 26, the lesion detection unit 27, the frame selection unit 29, the low-brightness region extraction unit 30, the GTC ratio calculation unit 31, the evaluation unit 32, and the body control unit 34 is configured by a central processing unit (CPU) and a control program for causing the CPU to execute various kinds of processing, but the processor 37 may be configured by using a field programmable gate array (FPGA), a digital signal processor (DSP), an application specific integrated circuit (ASIC), a graphics processing unit (GPU), or other integrated circuits (IC) or may be configured by a combination thereof.

Further, the image generation unit 21, the display control unit 22, the mammary gland region extraction unit 25, the thickness calculation unit 26, the lesion detection unit 27, the frame selection unit 29, the low-brightness region extraction unit 30, the GTC ratio calculation unit 31, the evaluation unit 32, and the body control unit 34 of the processor 37 can also be configured by being integrated partially or entirely into one CPU or the like.

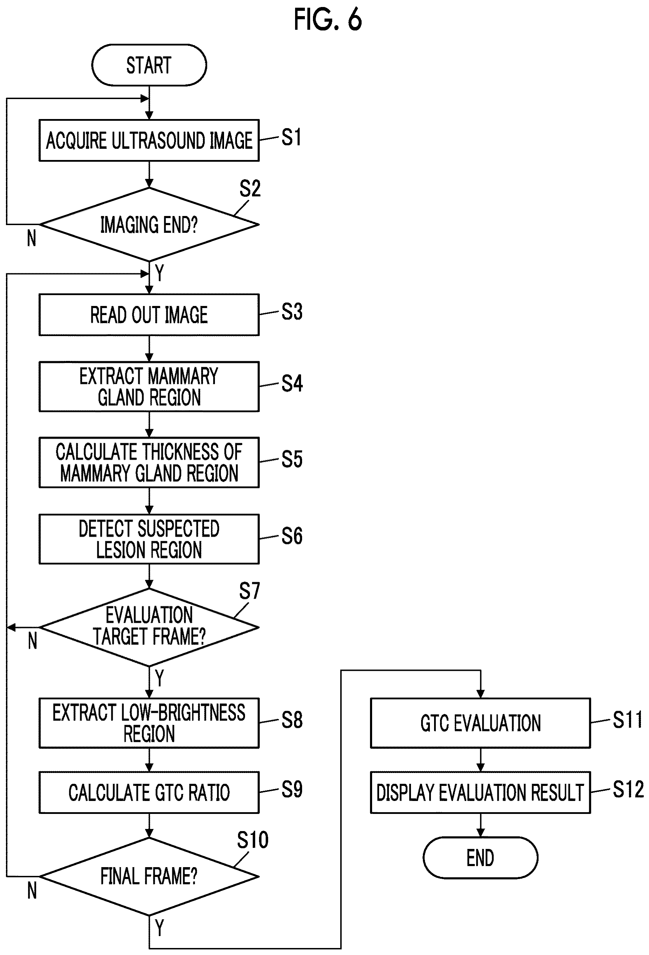

Hereinafter, an operation of the ultrasound diagnostic apparatus according to the embodiment will be described with reference to a flowchart shown in FIG. 6.

First, in step S1, the breast of the subject is imaged by using the ultrasound probe 1, and the ultrasound image U is acquired. In such a case, under the control of the body control unit 34, the transmission and reception of the ultrasound waves from the plurality of transducers of the transducer array 11 are started in accordance with the drive signal from the pulser 13 of the transmission and reception circuit 12 of the ultrasound probe 1, the ultrasound echo from the inside of the breast of the subject is received by the plurality of transducers of the transducer array 11, the reception signal which is an analog signal is output to the amplifying unit 14 and is amplified by the amplifying unit 14, and the amplified reception signal is AD-converted by the AD conversion unit 15 to acquire the reception data.

The reception focus processing is performed on the reception data by the beam former 16, and the sound ray signal generated by the reception focus processing is transmitted to the image generation unit 21 of the apparatus body 2, and as a result, the ultrasound image U representing the tomographic image information of the breast of the subject is generated by the image generation unit 21. In such a case, the signal processing unit 41 of the image generation unit 21 performs the correction of the attenuation in accordance with the depth of the reflection position of the ultrasound wave and the envelope detection processing on the sound ray signal, the DSC 42 performs the conversion into the image signal in accordance with the normal television signal scanning method, and the image processing unit 43 performs various types of necessary image processing such as gradation processing. The ultrasound image U acquired in this manner is stored in the image memory 24.

In step S2, the body control unit 34 determines whether or not to end the capturing of the ultrasound image U. The body control unit 34 can determine to end the capturing of the ultrasound image U, for example, in a case in which an instruction to end the capturing of the ultrasound image U is input by the user via the input device 35. In addition, the body control unit 34 can determine to continue the capturing of the ultrasound image U, for example, in a case in which the instruction to end the capturing of the ultrasound image U is not particularly input by the user via the input device 35.

In a case in which it is determined in step S2 to continue the capturing of the ultrasound image U, the processing returns to step S1, and a new ultrasound image U is acquired. As described above, as long as it is determined in step S2 to continue the capturing of the ultrasound image U, the processing of step S1 and the processing of step S2 are repeated, and the ultrasound images U of the plurality of frames are acquired. In such a case, the ultrasound images U of the plurality of frames are acquired by the user in a state in which the ultrasound probe 1 is moved on the breast of the subject.

In a case in which it is determined in step S2 to end the capturing of the ultrasound image U, the processing proceeds to step S3.

In step S3, the mammary gland region extraction unit 25 reads out the ultrasound image U of one frame, for example, the ultrasound image U of a first acquired frame among the ultrasound images U of the plurality of frames that are acquired by repeating step S1 and step S2 and stored in the image memory 24.

In step S4, the mammary gland region extraction unit 25 extracts the mammary gland region M from the ultrasound image U of the frame read out in step S3, and calculates the area of the extracted mammary gland region M. The mammary gland region extraction unit 25 can perform the image recognition using at least one of template matching, an image analysis technique using a feature value, such as AdaBoost, SVM, or SIFT, or a determination model that has been trained using a machine learning technique such as deep learning, in order to detect the breast region BR and to detect the mammary gland region M, for example.

In step S5, the thickness calculation unit 26 calculates the thickness of the mammary gland region M in the depth direction, which is extracted in step S4. For example, the thickness calculation unit 26 can calculate the maximum thickness of the mammary gland region M in the depth direction as the thickness of the mammary gland region M in the depth direction.

In step S6, the lesion detection unit 27 performs processing of detecting the suspected lesion region for the ultrasound image U of the frame read out in step S3. The lesion detection unit 27 can detect the suspected lesion region by using, for example, at least one of template matching, an image analysis technique using a feature value, such as AdaBoost, SVM, or SIFT, or a determination model that has been trained by using a machine learning technique such as deep learning. The determination model used here is a trained model that has learned a plurality of lesion parts in the ultrasound image U in which the breast region BR is imaged.

In step S7, in a case in which the frame read out in step S3 is a frame in which the thickness calculated in step S5 is equal to or greater than the predetermined thickness threshold value and is other than a frame in which the suspected lesion region is detected in step S6, the frame selection unit 29 selects the frame read out in step S3 as the frame of the evaluation target frame group.

Here, the frame determined to be the frame of the evaluation target frame group is a frame suitable for evaluating the GTC region, in which the thickness of the mammary gland region M is greater than the predetermined thickness and the suspected lesion region is not detected, and thus the GTC ratio can be accurately calculated.

In step S7, in a case in which the frame read out in step S3 is selected as the frame of the evaluation target frame group, the processing proceeds to step S8.

In step S8, the low-brightness region extraction unit 30 extracts the low-brightness region R1 having the brightness equal to or less than the predetermined brightness threshold value from the mammary gland region M extracted in step S4 for the ultrasound image U of the frame selected as the frame of the evaluation target frame group in step S7.

The low-brightness region extraction unit 30 can extract, for example, the pixel at which the brightness is equal to or less than the predetermined brightness threshold value from the mammary gland region M extracted by the mammary gland region extraction unit 25, and calculate the area occupied by the extracted pixel as the area of the low-brightness region R1 for each frame. The low-brightness region extraction unit 30 can also binarize the mammary gland region M extracted by the mammary gland region extraction unit 25 based on the predetermined brightness threshold value, and then use an algorithm such as a so-called watershed method, to extract the low-brightness region R1. The low-brightness region extraction unit 30 can also extract the low-brightness region R1 by using a determination model that has been trained by using a machine learning technique such as deep learning. As the determination model, for example, a trained model, which has learned the low-brightness region R1 in the mammary gland region M in the training ultrasound image in which the breast is imaged, is used.

In step S9, the GTC ratio calculation unit 31 calculates the GTC ratio based on the area of the mammary gland region M extracted in step S4 and the area of the low-brightness region R1 extracted in step S8, for the ultrasound image U of the frame selected as the frame of the evaluation target frame group in step S7. The GTC ratio calculation unit 31 can obtain the GTC ratio with high accuracy since the GTC ratio is calculated for the frames of the frame group of the evaluation target frame group.

In step S10, the body control unit 34 determines whether or not the frame read out in step S3 is a final frame among the plurality of frames acquired by repeating step S1 and step S2 and stored in the image memory 24. In a case in which it is determined that the frame read out in step S3 is not the final frame, the processing returns to step S3, and a new frame is read out from the image memory 24. Thereafter, the processing of steps S4 to the processing of S10 are performed.

In addition, even in a case in which the frame read out in step S3 is not selected as the frame of the evaluation target frame group in step S7, the processing returns to step S3, and a new frame is read out from the image memory 24. Thereafter, the processing of steps S4 to the processing of S7 are performed.

In this way, by repeating step S3 to step S7 and repeating step S3 to step S10, the evaluation target frame group is selected from the ultrasound images U of the plurality of frames stored in the image memory 24, and the extraction of the low-brightness region R1 and the calculation of the GTC ratio are performed for each of the selected evaluation target frame groups. As a result, while the user scans the entire breast of the subject using the ultrasound probe 1 in order to search for, for example, the suspected lesion region, the evaluation target frame group suitable for the evaluation of the GTC region is automatically selected, so that the user does not need to scan the breast of the subject again in order to evaluate the GTC region, and the evaluation target frame group suitable for the evaluation of the GTC region can be easily obtained.

In step S11, the evaluation unit 32 performs the GTC evaluation on the ultrasound image U of each frame of the evaluation target frame group, based on the GTC ratio calculated in step S9. The evaluation unit 32 can use, for example, an average value, a median value, or a sum value of the GTC ratio calculated in each of the evaluation target frame groups as the evaluation result of the GTC evaluation. For example, the evaluation unit 32 stores a predetermined breast cancer risk function for calculating a higher breast cancer risk value as the GTC ratio is lower, and can use the GTC ratio and the breast cancer risk value calculated based on the breast cancer risk function as the evaluation result of the GTC evaluation. In addition, the evaluation unit 32 can determine the category of the GTC region, such as Low or High, based on the GTC ratio, and use the category as the evaluation result of the GTC evaluation.

Finally, in step S12, the display control unit 22 displays the evaluation result regarding the GTC region obtained in step S11 on the monitor 23.

In a case in which the processing of step S12 is completed in this manner, the processing of the ultrasound diagnostic apparatus shown in FIG. 6 is completed.

As described above, with the ultrasound diagnostic apparatus according to Embodiment 1, the thickness calculation unit 26 calculates the thickness of the mammary gland region M in the depth direction for the ultrasound images U of the plurality of frames, the lesion detection unit 27 detects the suspected lesion region for the ultrasound images U of the plurality of frames, the frame selection unit 29 selects, as the evaluation target frame group, the frames which exclude the frame in which the suspected lesion region is detected, and in which the thickness of the mammary gland region M in the depth direction is equal to or greater than the thickness threshold value, and the evaluation unit 32 performs the GTC evaluation on the ultrasound image U of each frame of the evaluation target frame group. As a result, the user can easily perform the consideration of the risk of breast cancer based on the GTC region by automatically acquiring the evaluation target frame group suitable for the evaluation of the GTC region and the evaluation result of the GTC region for the evaluation target frame group, for example, by scanning the breast of the subject to search for the suspected lesion region without having to perform the work of evaluating the GTC region.

In addition, a case has been described in which the transmission and reception circuit 12 is provided in the ultrasound probe 1, but the transmission and reception circuit 12 may be provided in the apparatus body 2.

A case has been described in which the image generation unit 21 is provided in the apparatus body 2, but the image generation unit 21 may be provided in the ultrasound probe 1.

The apparatus body 2 may be a so-called stationary type, a portable type that is easy to carry, or a so-called handheld type that is configured by, for example, a smartphone or a tablet type computer. As described above, the type of the device constituting the apparatus body 2 is not particularly limited.

A case has been described in which the ultrasound probe 1 and the apparatus body 2 are connected to each other in a wired manner, but the ultrasound probe 1 and the apparatus body 2 may be connected to each other in a wireless manner.

In addition, in the flowchart of FIG. 6, after the ultrasound images U of the plurality of frames are acquired by repeating step S1 and step S2, the extraction of the mammary gland region M in step S4, the calculation of the thickness of the mammary gland region M in step S5, the detection of the suspected lesion region in step S6, and the selection of the frame of the evaluation target frame group in step S7 are performed, but the processing of step S4 to the processing of step S7 can be performed each time the ultrasound image U is acquired in step S1, and then the determination of step S2 can be performed.

In such a case, the mammary gland region M extracted in step S4, the thickness of the mammary gland region M calculated in step S5, the presence or absence of the suspected lesion region detected in step S6, and the information indicating whether or not the evaluation target frame group is selected in step S7 can be stored in the image memory 24 in association with the ultrasound image U acquired in step S1. In addition, in a case in which it is determined in step S2 to continue the capturing of the ultrasound image U, the processing returns to step S1, and then the processing of step S4 to the processing of step S7 are sequentially performed.

In addition, although it has been described that the processing of step S3 to the processing of step S10 is performed for each frame for the plurality of frames stored in the image memory 24, the processing of step S3 to the processing of step S9 can also be collectively performed for the plurality of frames. In such a case, in step S7, the frame selection unit 29 selects the evaluation target frame group from the plurality of frames stored in the image memory 24. In step S8 and step S9 after step S7, the processing of extracting the low-brightness region R1 and calculating the GTC ratio is performed on the selected evaluation target frame group.

In addition, the apparatus body 2 can also comprise a fat region detection unit (not shown) that detects a fat region in the mammary gland region M from the ultrasound image U and that calculates an area of the detected fat region. The fat region detection unit can perform the processing of detecting the fat region by using, for example, at least one of template matching, an image analysis technique using a feature value, such as AdaBoost, SVM, or SIFT, or a determination model that has been trained by using a machine learning technique such as deep learning. The determination model used here is a trained model that has learned a plurality of fat regions in the ultrasound image U in which the breast region BR is imaged.

In such a case, the frame selection unit 29 can select, as the evaluation target frame group, frames which exclude the frame in which the suspected lesion region is detected by the lesion detection unit 27 and the frame in which the fat region having the area equal to or greater than a fat region area is detected by the fat region detection unit, and in which the thickness of the mammary gland region M in the depth direction, which is calculated by the thickness calculation unit 26, is equal to or greater than the thickness threshold value, among the ultrasound images U of the plurality of frames generated by the image generation unit 21.

Further, the frame selection unit 29 can exclude frames at regular intervals in time series from the plurality of frames generated by the image generation unit 21, and select the evaluation target frame group from the remaining frame groups. As a result, the number of frames of the evaluation target frame group is reduced, and the calculation load and the power consumption required for the subsequent processing in the low-brightness region extraction unit 30, the GTC ratio calculation unit 31, and the like can be reduced.

### [Embodiment 2]

In a case in which the ultrasound probe 1 is not sufficiently close to the body surface of the subject or in a case in which the ultrasound probe 1 is separated from the body surface of the subject, the ultrasound wave transmitted from the transducer array 11 is radiated into the air, and a so-called shadow known as an artifact of a stripe pattern extending in the depth direction may be depicted in the ultrasound image U. The ultrasound diagnostic apparatus can also perform the shadow detection processing, and select the evaluation target frame group in consideration of the shadow detection result.

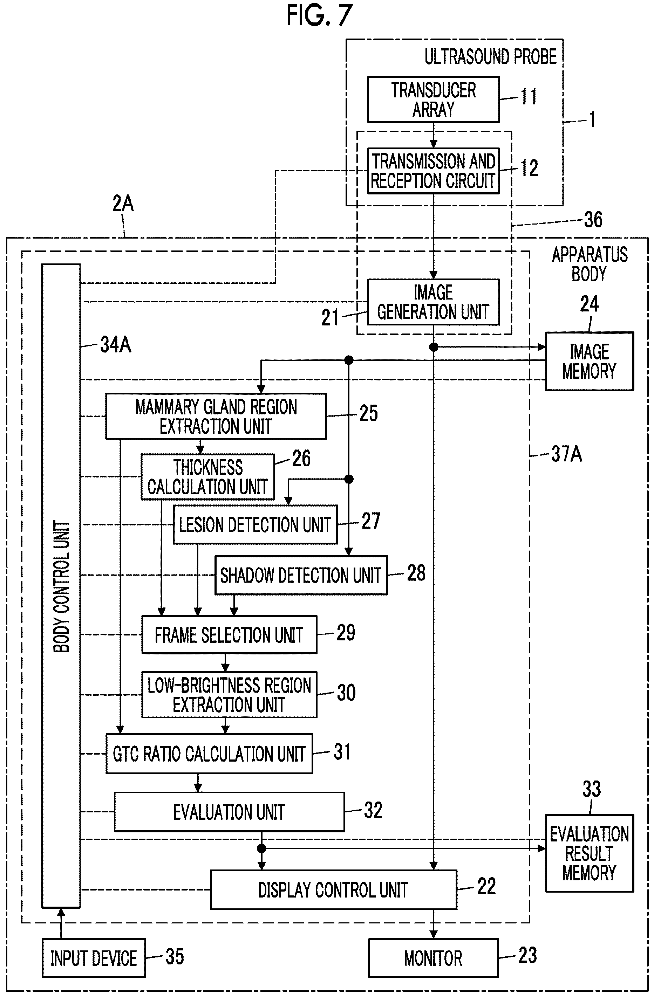

FIG. 7 shows a configuration of an ultrasound diagnostic apparatus according to Embodiment 2. The ultrasound diagnostic apparatus according to Embodiment 2 comprises an apparatus body 2A instead of the apparatus body 2, as compared to the ultrasound diagnostic apparatus according to Embodiment 1 shown in FIG. 1. The apparatus body 2A further comprises a shadow detection unit 28 and comprises a body control unit 34A instead of the body control unit 34, as compared to the apparatus body 2 according to Embodiment 1.

In the apparatus body 2A, the shadow detection unit 28 is connected to the image memory 24. The shadow detection unit 28 is connected to the frame selection unit 29 and the body control unit 34A. In addition, the image generation unit 21, the display control unit 22, the mammary gland region extraction unit 25, the thickness calculation unit 26, the lesion detection unit 27, the shadow detection unit 28, the frame selection unit 29, the low-brightness region extraction unit 30, the GTC ratio calculation unit 31, the evaluation unit 32, and the body control unit 34 constitute a processor 37A for the apparatus body 2A.

The shadow detection unit 28 detects the shadow for each of the ultrasound images U of the plurality of frames. For example, the shadow detection unit 28 can create one-dimensional brightness data in a lateral direction obtained by integrating brightness in the depth direction at each position in the lateral direction perpendicular to the depth direction, and determine that the shadow is present in the ultrasound image U in a case in which a length in the lateral direction of a range having an integrated value of the brightness equal to or less than a brightness integration threshold value determined in the one-dimensional brightness data is equal to or greater than a predetermined length.

In addition, the shadow detection unit 28 can also detect the shadow using, for example, at least one of template matching, an image analysis technique using a feature amount such as AdaBoost, SVM, or SIFT, or a determination model that has been trained using a machine learning technique such as deep learning. The determination model used here is a trained model that has learned a plurality of shadows in the ultrasound image U in which the breast region BR is imaged.

The frame selection unit 29 selects, as the evaluation target frame group, frames which exclude the frame in which the suspected lesion region is detected by the lesion detection unit 27 and the frame in which the shadow is detected by the shadow detection unit 28, and in which the thickness of the mammary gland region M in the depth direction, which is calculated by the thickness calculation unit 26, is equal to or greater than the thickness threshold value.

The low-brightness region extraction unit 30 extracts the low-brightness region R1 for the ultrasound image U of the evaluation target frame group selected by the frame selection unit 29.

The GTC ratio calculation unit 31 calculates the GTC ratio based on the area of the mammary gland region M and the area of the low-brightness region R1.

The evaluation unit 32 performs the GTC evaluation based on the GTC ratio.

Here, in a case in which the ultrasound image U includes the shadow, a region narrower than the original low-brightness region R1 may be detected as the low-brightness region R1 due to the overlap of the shadow with the low-brightness region R1. In Embodiment 2, the evaluation target frame group selected by the frame selection unit 29 excludes the ultrasound image U in which the shadow is detected by the shadow detection unit 28, so that the low-brightness region extraction unit 30 can detect the low-brightness region R1 having the original area in the ultrasound image U. Therefore, the GTC ratio calculation unit 31 can accurately calculate the GTC ratio, and the evaluation unit 32 can output the result of the GTC evaluation with high accuracy.

As described above, with the ultrasound diagnostic apparatus according to Embodiment 2, the shadow detection unit 28 detects the shadow for each of the ultrasound images U of the plurality of frames, and the frame selection unit 29 selects, as the evaluation target frame group, frames which exclude the frame in which the suspected lesion region is detected by the lesion detection unit 27 and the frame in which the shadow is detected by the shadow detection unit 28, and in which the thickness of the mammary gland region M in the depth direction, which is calculated by the thickness calculation unit 26, is equal to or greater than the thickness threshold value, so that the accuracy of the GTC evaluation is improved, and the user can further accurately consider the risk of breast cancer of the subject.

### [Embodiment 3]

Although it has been described that the evaluation unit 32 evaluates the GTC region based on the GTC ratio calculated by the GTC ratio calculation unit 31, the method of evaluation using the evaluation unit 32 is not limited to this.

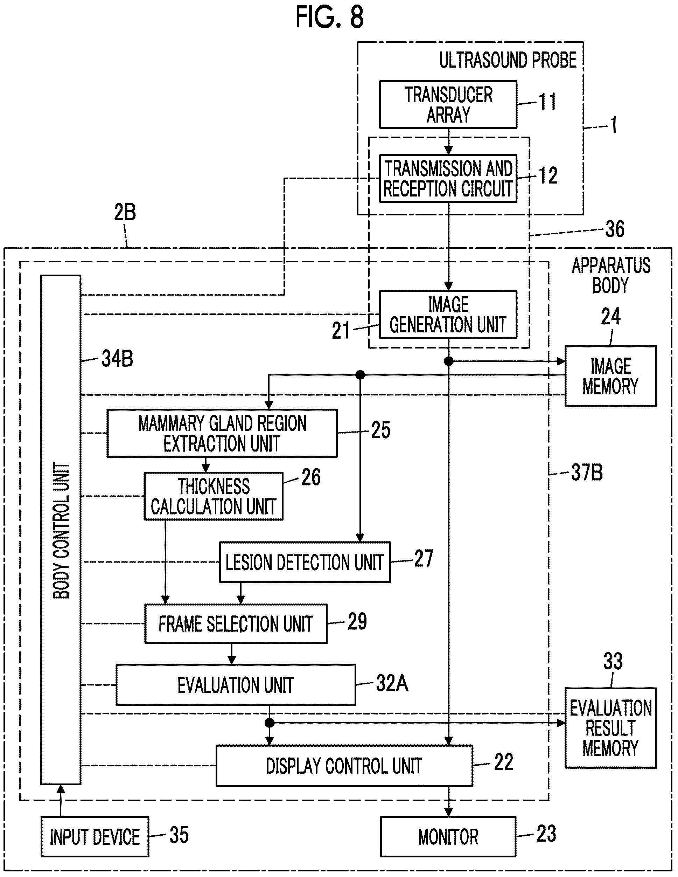

FIG. 8 shows a configuration of an ultrasound diagnostic apparatus according to Embodiment 3. The ultrasound diagnostic apparatus according to Embodiment 3 comprises an apparatus body 2B instead of the apparatus body 2, as compared to the ultrasound diagnostic apparatus according to Embodiment 1 shown in FIG. 1. The apparatus body 2B is obtained by excluding the low-brightness region extraction unit 30 and the GTC ratio calculation unit 31, comprising an evaluation unit 32B instead of the evaluation unit 32, and comprising a body control unit 34B instead of the body control unit 34, as compared to the apparatus body 2 in Embodiment 1.

In the apparatus body 2B, the evaluation unit 32B is connected to the frame selection unit 29. In addition, the image generation unit 21, the display control unit 22, the mammary gland region extraction unit 25, the thickness calculation unit 26, the lesion detection unit 27, the frame selection unit 29, the evaluation unit 32B, and the body control unit 34B constitute a processor 37B for the apparatus body 2B.

The evaluation unit 32B can determine the category of the GTC region in the evaluation target frame group using a trained model that has been trained through machine learning based on a plurality of pieces of training data each of which includes the ultrasound image U in which the mammary gland region M as shown in FIG. 4 or FIG. 5 is imaged and the category of the GTC region in the ultrasound image U, and can output the determined category as the evaluation result. The association between the ultrasound image U and the category of the GTC region in the training data can be performed by an expert, such as a skilled doctor.

Even in a case in which the category of the GTC region is determined based on the ultrasound image U of the evaluation target frame group by the learning model in the machine learning as in the ultrasound diagnostic apparatus according to Embodiment 3, the evaluation target frame group suitable for the evaluation of the GTC region is selected by the frame selection unit 29, so that the user can easily perform the consideration of the risk of breast cancer based on the GTC region by automatically acquiring the evaluation target frame group suitable for the evaluation of the GTC region and the evaluation result of the GTC region for the evaluation target frame group, for example, by scanning the breast of the subject to search for the suspected lesion region without having to perform the work of evaluating the GTC region.

The ultrasound diagnostic apparatus according to Embodiment 3 can be configured by excluding the frame selection unit 29 and the low-brightness region extraction unit 30 from the ultrasound diagnostic apparatus according to Embodiment 1, but can also be configured by excluding the frame selection unit 29 and the low-brightness region extraction unit 30 from the ultrasound diagnostic apparatus according to Embodiment 2.

### [Embodiment 4]

In a case in which the user stops the movement of the ultrasound probe 1 in the middle of the acquisition of the ultrasound images U of the plurality of frames or in a case in which the movement speed of the ultrasound probe 1 by the user is extremely small, the ratio of the frames captured at a specific portion in the evaluation target frame group may be increased. Therefore, the ultrasound diagnostic apparatus can eliminate the bias of the imaging portion of the ultrasound image U by excluding the frames that are adjacent in time series and that has a small change in the value of the GTC ratio in the evaluation target frame group.

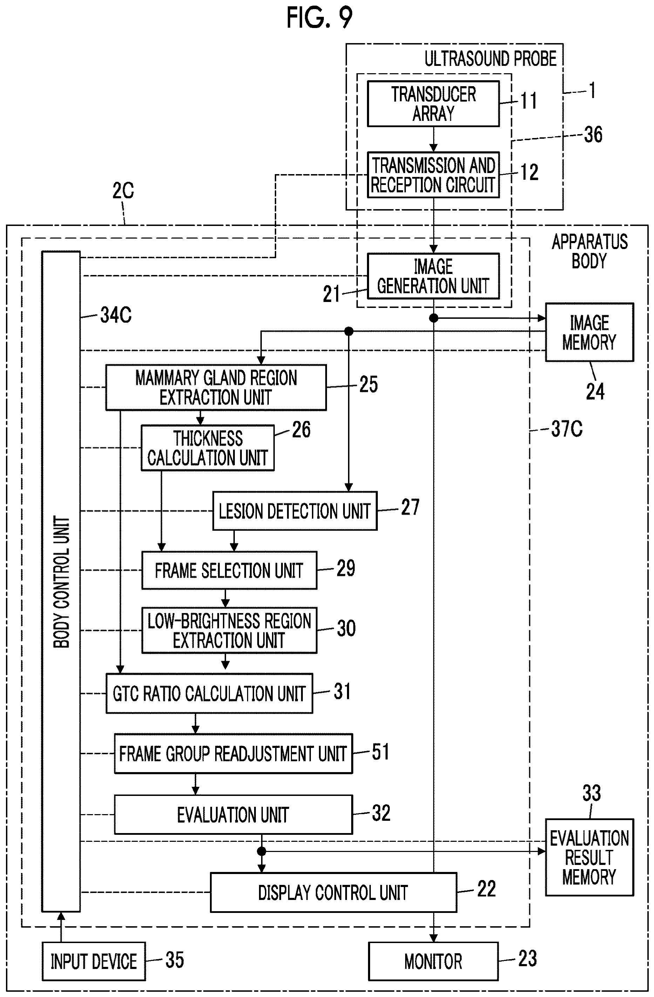

FIG. 9 shows a configuration of an ultrasound diagnostic apparatus according to Embodiment 4. The ultrasound diagnostic apparatus according to Embodiment 4 comprises an apparatus body 2C instead of the apparatus body 2, as compared to the ultrasound diagnostic apparatus according to Embodiment 1 shown in FIG. 1. The apparatus body 2C further comprises a frame group readjustment unit 51 and comprises a body control unit 34C instead of the body control unit 34, as compared to the apparatus body 2 according to Embodiment 1.

In the apparatus body 2C, the frame group readjustment unit 51 is connected to the GTC ratio calculation unit 31. The evaluation unit 32 and the body control unit 34C are connected to the frame group readjustment unit 51. In addition, the image generation unit 21, the display control unit 22, the mammary gland region extraction unit 25, the thickness calculation unit 26, the lesion detection unit 27, the frame selection unit 29, the low-brightness region extraction unit 30, the evaluation unit 32, the body control unit 34C, and the frame group readjustment unit 51 constitute a processor 37C for the apparatus body 2C.

The frame group readjustment unit 51 has a predetermined ratio threshold value for a difference in the GTC ratio, and in a case in which the GTC ratio in one frame is equal to or less than the ratio threshold value for the GTC ratio in an immediately preceding frame in time series in the evaluation target frame group, that is, a difference between the GTC ratio in one frame in the evaluation target frame group and the GTC ratio in the frame immediately preceding the one frame in time series is equal to or less than the ratio threshold value, readjusts the evaluation target frame group such that one frame is excluded.

As a result, for example, it is possible to prevent the bias of the imaging portion of the ultrasound image U, which is caused by the user stopping the movement of the ultrasound probe 1 or the very small movement speed of the ultrasound probe 1 by the user, so that it is possible to prevent the value of the GTC ratio calculated for the evaluation target frame group from being biased to a value within a certain range.

The evaluation unit 32 stores the GTC ratio in the evaluation target frame group readjusted by the frame group readjustment unit 51 in the evaluation result memory 33 as the result of the GTC evaluation and displays the GTC ratio on the monitor 23. Since the GTC ratio in the readjusted evaluation target frame group is prevented from being biased to a value within a certain range, the user, such as the doctor, can accurately consider the risk of breast cancer for the subject with reference to the evaluation result displayed on the monitor 23.

As described above, with the ultrasound diagnostic apparatus according to Embodiment 4, the evaluation target frame group is readjusted by the frame group readjustment unit 51 such that the value of the GTC ratio does not deviate from a certain range of values, so that the user can accurately consider the risk of breast cancer for the subject.

Although it has been described that the GTC ratio in the readjusted evaluation target frame group is stored in the evaluation result memory 33 as the evaluation result of the GTC region and is displayed on the monitor 23, the breast cancer risk value can also be calculated based on the GTC ratio in the readjusted evaluation target frame group, and the category of the GTC region can also be output.

In addition, the ultrasound diagnostic apparatus according to Embodiment 4 can be configured by adding the frame group readjustment unit 51 to the ultrasound diagnostic apparatus according to Embodiment 1, but can also be configured by adding the frame group readjustment unit 51 to the ultrasound diagnostic apparatus according to Embodiment 2.

### [Embodiment 5]

The ultrasound diagnostic apparatus can create a histogram of the GTC ratio calculated for the evaluation target frame group and readjust the evaluation target frame group based on the created histogram.

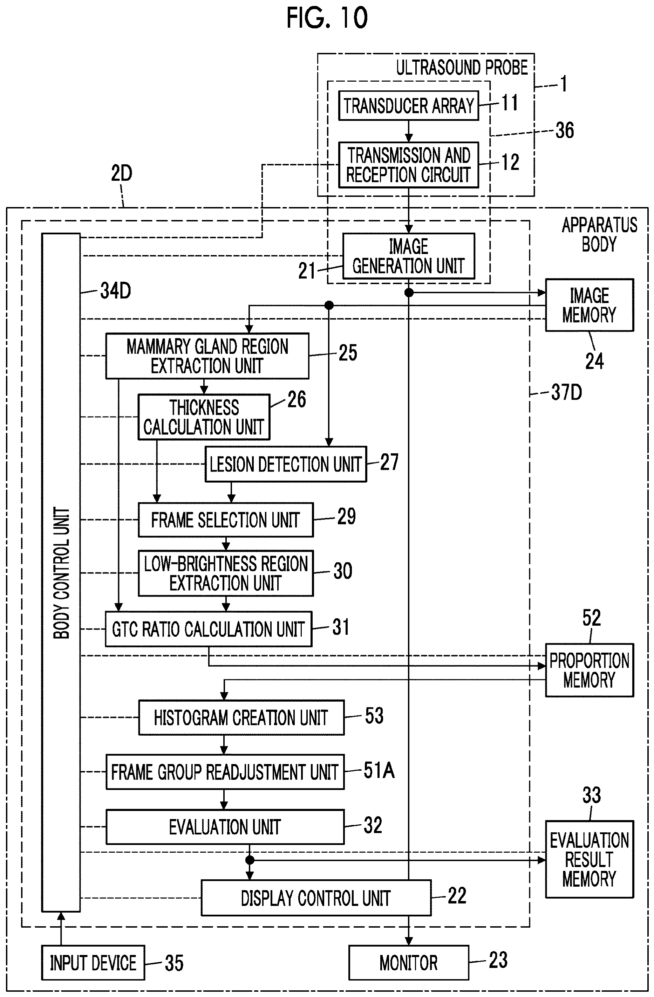

FIG. 10 shows a configuration of an ultrasound diagnostic apparatus according to Embodiment 5. The ultrasound diagnostic apparatus according to Embodiment 5 comprises an apparatus body 2D instead of the apparatus body 2, as compared to the ultrasound diagnostic apparatus according to Embodiment 1 shown in FIG. 1. The apparatus body 2D further comprises a frame group readjustment unit 51A, a ratio memory 52, and a histogram creation unit 53, and comprises a body control unit 34D instead of the body control unit 34, as compared to the apparatus body 2 according to Embodiment 1.

In the apparatus body 2D, the ratio memory 52 is connected to the GTC ratio calculation unit 31. The histogram creation unit 53, the frame group readjustment unit 51A, and the evaluation unit 32 are connected sequentially to the ratio memory 52. In addition, the ratio memory 52, the histogram creation unit 53, and the frame group readjustment unit 51A are connected to the body control unit 34D. In addition, the image generation unit 21, the display control unit 22, the mammary gland region extraction unit 25, the thickness calculation unit 26, the lesion detection unit 27, the frame selection unit 29, the low-brightness region extraction unit 30, the GTC ratio calculation unit 31, the evaluation unit 32, the body control unit 34D, the frame group readjustment unit 51A, and the histogram creation unit 53 constitute a processor 37D for the apparatus body 2D.

The ratio memory 52 is a memory which stores the GTC ratio calculated by the GTC ratio calculation unit 31. As the ratio memory 52, for example, recording media such as a flash memory, an HDD, an SSD, an FD, an MO disc, an MT, an RAM, a CD, a DVD, an SD card, and an USB memory can be used.

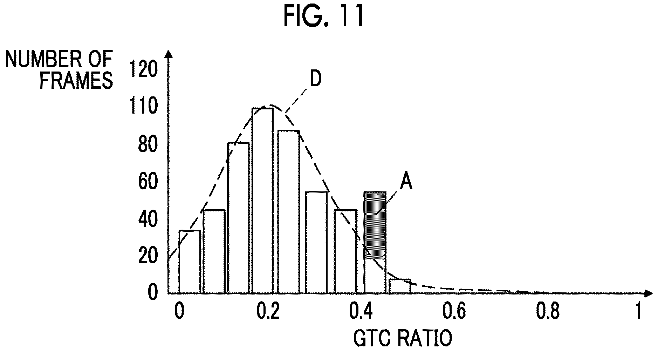

For example, as shown in FIG. 11, the histogram creation unit 53 creates the histogram of the GTC ratio in the plurality of frames in the evaluation target frame group stored in the ratio memory 52. In this histogram, a class on a horizontal axis is a range of the GTC ratio value, and a frequency on a vertical axis is the number of frames.

For example, the frame group readjustment unit 51A can calculate an average and a variance with respect to the GTC ratio in the plurality of frames in the evaluation target frame group, and calculate a predetermined distribution D, such as a Gaussian distribution, as shown in FIG. 11 based on the calculated average and variance. The frame group readjustment unit 51A readjusts the evaluation target frame group such that the frame corresponding to the GTC ratio that appears with a higher frequency than the predetermined distribution D is excluded in the histogram created by the histogram creation unit 53. In the example of FIG. 11, the GTC ratio belonging to a class A appears with a higher frequency than the distribution D. In such a case, the frame group readjustment unit 51A can exclude a part of the frames corresponding to the class A and readjust the evaluation target frame group such that the GTC ratio belonging to the class A is represented with a lower frequency than the distribution D.

The evaluation unit 32 stores the GTC ratio in the evaluation target frame group readjusted by the frame group readjustment unit 51A in the evaluation result memory 33 as the result of the GTC evaluation and displays the GTC ratio on the monitor 23.

As described above, with the ultrasound diagnostic apparatus according to Embodiment 5, the frame group readjustment unit 51A readjusts the evaluation target frame group such that the frame corresponding to the GTC ratio that appears with a higher frequency than the predetermined distribution D is excluded, based on the histogram created by the histogram creation unit 53, so that the user can accurately consider the risk of breast cancer for the subject by using the GTC ratio calculated for the readjusted evaluation target frame group.

Although it has been described that the GTC ratio in the readjusted evaluation target frame group is stored in the evaluation result memory 33 as the evaluation result of the GTC region and is displayed on the monitor 23, the breast cancer risk value can also be calculated based on the GTC ratio in the readjusted evaluation target frame group, and the category of the GTC region can also be output.

In addition, the ultrasound diagnostic apparatus according to Embodiment 5 can be configured by adding the frame group readjustment unit 51A, the ratio memory 52, and the histogram creation unit 53 to the ultrasound diagnostic apparatus according to Embodiment 1, but can also be configured by adding the frame group readjustment unit 51A, the ratio memory 52, and the histogram creation unit 53 to the ultrasound diagnostic apparatus according to Embodiment 2.

### Explanation of References

1: ultrasound probe
2, 2A, 2B, 2C, 2D: apparatus body
11: transducer array
12: transmission and reception circuit
13: pulser
14: amplifying unit
15: AD conversion unit
16: beam former
21: image generation unit
22: display control unit
23: monitor
24: image memory
25: mammary gland region extraction unit
26: thickness calculation unit
27: lesion detection unit
28: shadow detection unit
29: frame selection unit
30: low-brightness region extraction unit
31: GTC ratio calculation unit
32, 32A: evaluation unit
33: evaluation result memory
34, 34A, 34B, 34C, 34D: body control unit
35: input device
36: image acquisition unit
37, 37A, 37B, 37C, 37D: processor
41: signal processing unit
42: DSC
43: image processing unit
51, 51A: frame group readjustment unit
52: ratio memory
53: histogram creation unit
A: class
D: distribution
BR: breast region
L1: front boundary line
L2: rear boundary line
M: mammary gland region
R1: low-brightness region
R2: edematous stroma
S: skin
T: pectoralis major
U: ultrasound image

## Claims

1. An ultrasound diagnostic apparatus comprising:
an ultrasound probe;
an image acquisition unit that performs a scan using the ultrasound probe to continuously acquire ultrasound images of a plurality of frames in which a breast of a subject is imaged;
a mammary gland region extraction unit that extracts a mammary gland region from each of the ultrasound images of the plurality of frames and that calculates an area of the mammary gland region;
a thickness calculation unit that calculates a thickness of the mammary gland region in a depth direction, which is extracted by the mammary gland region extraction unit, for each of the ultrasound images of the plurality of frames;
a lesion detection unit that detects a suspected lesion region for each of the ultrasound images of the plurality of frames;
a frame selection unit that selects, as an evaluation target frame group, at least frames which exclude a frame in which the suspected lesion region is detected by the lesion detection unit, and in which the thickness of the mammary gland region in the depth direction, which is calculated by the thickness calculation unit, is equal to or greater than a predetermined thickness threshold value, among the plurality of frames; and
an evaluation unit that performs a glandular tissue component evaluation on the ultrasound image of each frame of the evaluation target frame group.

2. The ultrasound diagnostic apparatus according to claim 1, further comprising:
a shadow detection unit that detects a shadow for each of the ultrasound images of the plurality of frames,
wherein the frame selection unit selects, as the evaluation target frame group, frames which exclude the frame in which the suspected lesion region is detected by the lesion detection unit and a frame in which the shadow is detected by the shadow detection unit, and in which the thickness of the mammary gland region in the depth direction, which is calculated by the thickness calculation unit, is equal to or greater than the predetermined thickness threshold value.

3. The ultrasound diagnostic apparatus according to claim 1, further comprising:
an evaluation result memory that stores an evaluation result obtained by the evaluation unit;
a monitor; and
a display control unit that displays the ultrasound image and the evaluation result on the monitor.

4. The ultrasound diagnostic apparatus according to claim 3, further comprising:
a low-brightness region extraction unit that extracts a low-brightness region having brightness equal to or less than a predetermined brightness threshold value from the mammary gland region extracted by the mammary gland region extraction unit for the ultrasound image of the evaluation target frame group; and
a glandular tissue component ratio calculation unit that calculates a glandular tissue component ratio based on the area of the mammary gland region extracted by the mammary gland region extraction unit for the ultrasound image of the evaluation target frame group and an area of the low-brightness region extracted by the low-brightness region extraction unit,
wherein the evaluation unit performs the glandular tissue component evaluation based on the glandular tissue component ratio calculated by the glandular tissue component ratio calculation unit.

5. The ultrasound diagnostic apparatus according to claim 4, further comprising:
a frame group readjustment unit that, in a case in which a difference between the glandular tissue component ratio in one frame in the evaluation target frame group and the glandular tissue component ratio in a frame immediately preceding the one frame is equal to or less than a predetermined ratio threshold value, readjusts the evaluation target frame group such that the one frame is excluded,
wherein the evaluation unit stores the glandular tissue component ratio in the evaluation target frame group readjusted by the frame group readjustment unit in the evaluation result memory as a result of the glandular tissue component evaluation, and displays the glandular tissue component ratio on the monitor.

6. The ultrasound diagnostic apparatus according to claim 4, further comprising:
a ratio memory that stores the glandular tissue component ratio calculated by the glandular tissue component ratio calculation unit;
a histogram creation unit that creates a histogram of the glandular tissue component ratio in a plurality of frames in the evaluation target frame group stored in the ratio memory; and
a frame group readjustment unit that readjusts the evaluation target frame group such that a frame corresponding to the glandular tissue component ratio that appears with a higher frequency than a distribution determined in the histogram is excluded,
wherein the evaluation unit stores the glandular tissue component ratio in the evaluation target frame group readjusted by the frame group readjustment unit in the evaluation result memory as a result of the glandular tissue component evaluation, and displays the glandular tissue component ratio on the monitor.

7. The ultrasound diagnostic apparatus according to any one of claims 1 to 6,
wherein the frame selection unit excludes the frames at regular intervals in time series from the plurality of frames, and selects the evaluation target frame group from remaining frame groups.

8. A method of controlling an ultrasound diagnostic apparatus, the method comprising:
performing a scan using an ultrasound probe to continuously acquire ultrasound images of a plurality of frames in which a breast of a subject is imaged;
extracting a mammary gland region from each of the ultrasound images of the plurality of frames;
calculating a thickness of the extracted mammary gland region in a depth direction for each of the ultrasound images of the plurality of frames;
detecting a suspected lesion region for each of the ultrasound images of the plurality of frames;
selecting, as an evaluation target frame group, at least frames which exclude a frame in which the suspected lesion region is detected, and in which the calculated thickness of the mammary gland region in the depth direction is equal to or greater than a predetermined thickness threshold value, among the plurality of frames; and
performing a glandular tissue component evaluation on the ultrasound image of each frame of the evaluation target frame group.
